# EUROPEAN PATENT SPECIFICATION

(11) **EP 0 703 244 B1**
(45) Date of publication and mention of the grant of the patent: **15.12.1999**
(21) Application number: 94115132.6
(22) Date of filing: 26.09.1994
(51) Int. Cl.: C08F 2/32, A61L 15/00

(54) **Process for the manufacture of a superabsorption acrylic polymer**
Verfahren zur Herstellung eines superabsorbierenden Acrylpolymer
Procédé de préparation d'un polymère acrylique super absorbant

(43) Date of publication of application: 27.03.1996
(73) Proprietor: MELAMIN Kemicna tovarna d.d. Kocevje, 61330 Kocevje (SI)
(72) Inventor: Ogorelec, Primoz, SI-61330 Kocevje (SI)
(74) Representative: Viering, Jentschura & Partner

(56) References cited:
- GB-A- 2 242 684

## Description

### Technical Field

The present invention belongs to the field of organic-chemical synthesis of artificial polymers and relates to a process for the manufacture of a superabsorption acrylic polymer by suspension polymerization applicable in sanitary products.

### Technical Problem

Superabsorption acrylic type polymers and their use in sanitary products for the binding of water and water liquids have been known for a long time. It is stated in numerous patent literature (GB 2,088,392 A, US 4,093,776, US 4,342,858 and US 2,982,749) that they are manufactured by a process of solution polymerization. This process is quite useful in the synthesis of superabsorbents and gives products in the form of crushed pieces, however, it requires a rather intensive work owing to the nature of the product and, additionally, it is less convenient for automation.

Therefore there existed a need for an improved process for the manufacture of a superabsorption acrylic type polymer by suspension polymerization, which would make possible the manufacture of polymeric products in a form suitable for the incorporation into sanitary products, i.e. in granulated form, and also allow a good control of the polymerization parameters and a re-use of the solvent as the continuous phase.

### Prior art

Superabsorption acrylic type polymers have been commercially available for several years. Their use in sanitary products such as disposable diapers has been known for nearly ten years. Superabsorption acrylic polymers belong to the group of hydrogels, i.e. water-retaining gels, which are water insoluble polymers with a high hydrophility resulting from hydrophilic groups such as -CONH₂, -COOH and -COONa present in the main chain. The high-molecular linear chain is sparsely cross-linked with covalent bonds but, additionally, with hydrogen bonds and Van der Walls forces. Water may freely enter into the polymer structure, which swells up to a volume equilibrium. Superabsorption acrylic type polymers possess a high absorptivity for the physiologic solution (up to 50 ml/g), the pH of the given-off water is from 5.5 to 7, their content of the monomers is under 1000 ppm, mostly under 500 ppm, and they are commercially available in a white granulate form and correspond to sanitary standards.

All commercially available superabsorbents known to us are made according to the solution polymerization process, which is evident from the particles occurring in the form of the crushed pieces of crystalline appearance. For the incorporation into sanitary products, however, superabsorbents in the form of flowable granules having a suitable granularity, generally from 0.2 to 1 mm, are required. Further, in the solution polymerization process the continuous phase, i.e. organic solvent or solvent mixture, remains as a waste upon filtering off the superabsorbent. The recycling of such a solvent is very difficult due to the contamination with emulsifier, initiator and micronized or submicronized polymer particles. Therefore such manufacture is uneconomic.

### Technical Solution

The present invention relates to a process for the manufacture of a superabsorption acrylic polymer on the basis of sodium acrylate-acrylamide copolymer using the recycled continuous phase obtained by the below-described process when the polymer is filtered off.

Of particular relevance for the manufacture of the superabsorption polymers are acrylic polymers, especially those containing as the basic monomer acrylic or methacrylic acid that, as a rule, have been previously partly or entirely neutralised with alkali hydroxides, and esters and amides thereof, particularly acrylamide. The polymerization mainly takes place according to the chain mechanism with free radicals and normally in a water medium. Usually, the initiators are inorganic peroxo compounds, azo compounds and redox systems.

The process for the manufacture of a superabsorption acrylic polymer on the basis of sodium acrylate-acrylamide copolymer is performed in a hydrophobic organic solvent representing the continuous phase. After mechanical separation this continuous phase is re-used in the following charge as the basis of the continuous phase. The mechanical separation is performed in such a manner that the residual polymer particles are used as polymerization seeds at the new addition of a mixture of the monomers in the new charge. Since the mixture of the monomers is made in a water solution, the residual particles of superabsorbent also act as an absorbent for the water solution so that the monomer mixture is absorbed into the particles and the polymerization takes place in the gel practically from the beginning. Due to the gel effect, preferably a higher molecular weight is formed, which has an advantageous influence upon the properties of the obtained superabsorbent.

Therefore the originality of the present process lies in the recycling of the solvent and in the use of the residual superabsorbent particles in the filtrate as a medium for absorbing the mixture of the monomers so that the polymerization with gel effect is obtained immediately.

The present invention is illustrated by the following two examples.

### Example 1

In a three-necked glass flask equipped with a thermometer and a tube for blowing in nitrogen, sodium hydroxide (20 g) was dissolved in water (60 ml). Acrylic acid (about 48 g) was added carefully until a pH 6.3 to 7.0 was achieved. Subsequently, acrylamide (24 g) was added to the mixture. It was cooled to a temperature under 30 °C and then potassium persulphate (29 mg) and sodium bisulphite (50 mg) were added. The resulting mixture was poured into a dropping funnel.

In a glass laboratory reactor equipped with an anchor stirrer, a tube for blowing in nitrogen, a dropping funnel, a thermometer and an attachment for separating water connected with a reflux condenser, the emulsifier Etolate 24 A30 (Teol, Ljubljana) (4 g) and ethylcellulose (6 g) in toluene (520 g) were dissolved. The velocity of the stirrer was set at 250 revolutions per minute. The mixture of monomers was added to the reactor for one hour at the temperature of 50 °C.

20 minutes after the dropping had been finished, the temperature of the bath was raised and the reactor mixture was distilled azeotropically. Water was separated. When 100 ml of water were distilled off, the reaction mixture was cooled and filtered. The filtered-off polymer was washed with pure toluene and dried in a dryer at the temperature of 110 °C. The filtrate was saved.

The resulting polymer had an absorption for the physiologic solution over 45 ml/mg, a neutral to slightly acidic pH and the content of monomers under 100 ppm. After filtration it was in the form of flowable agglomerates.

### Example 2

In a three-necked glass flask equipped with a thermometer and a tube for blowing in nitrogen, sodium hydroxide (20 g) was dissolved in water (60 ml). Acrylic acid (about 48 g) was added carefully until a pH 6.3 to 7.0 was achieved. Subsequently, acrylamide (20 g) was added to the mixture. The mixture was blown through with nitrogen, cooled to a temperature under 25 °C and then potassium persulphate (29 mg) and sodium bisulphite (70 mg) were added. The resulting mixture was poured into a dropping funnel.

In a glass laboratory reactor equipped with an anchor stirrer, a tube for blowing in nitrogen, a dropping funnel, a thermometer and an attachment for separating water connected with reflux condenser, the emulsifier Etolate 24 A30 (Teol, Ljubljana) (3 g) and ethylcellulose (6 g) in the filtrate from Example 1 were added. Then it was blown through with nitrogen. Subsequently, such an amount of toluene was added that the volume of the toluene solution was 700 ml. The speed of the stirrer was set at 300 revolutions per minute. The mixture of monomers was added to the reactor for one hour at the temperature of 45 °C.

20 minutes after the dropping had been finished, the temperature of the bath was raised and the reactor mixture was distilled azeotropically. Water was separated. When 90 ml of water were distilled off, the reaction mixture was cooled and filtered. The filtered-off polymer was washed with pure toluene and dried in a dryer at the temperature of 110 °C.

The resulting polymer had an absorption for the physiologic solution over 50 ml/mg, a neutral to slightly acidic pH and the content of monomers under 100 ppm. After filtration it was in the form of flowable agglomerates.

In Example 2 the continuous phase of example 1 from which the polymer had been filtered off was used as the continuous phase. Equally, the continuous phase of Example 2 after the filtration could be re-used in further syntheses.

Other aromatic and cycloaliphatic hydrophobic solvents such as xylene, cyclohexane and benzene, chlorinated hydrocarbons as well as aliphatic solvents such as hexane, heptane, octane etc. can be used as well, however, it is necessary to adapt the emulsifier and the protecting colloid to the solvent.

## Claims

1. Process for the manufacture of a superabsorption acrylic polymer in the form of flowable granules, characterized in that sodium acrylate and acrylamide are subjected to one-stage polymerization in a w/o type suspension in a temperature range from 40°C to 60°C, wherein, as the continuous phase, there is used a filtrate obtained after filtering off the polymer from the said polymerization carried out with a hydrophobic organic solvent as the continuous phase, and wherein the filtering is performed in such a manner that polymer particles remain in the filtrate as polymerization seeds.

## Patentansprüche

1. Verfahren zur Herstellung eines superabsorbierenden Acrylpolymers in der Form von fließbaren Körnchen, dadurch gekennzeichnet, daß Natriumacrylat und Acrylamid einer einstufigen Polymerisation in einer Suspension des Wasser-Ol-Typus in einem Temperaturbereich von 40°C bis 60°C unterworfen werden, worin als die kontinuierliche Phase das nach der Abfiltrierung des Polymers aus der erwähnten, mit einem hydrophoben, als kontinuierliche Phase dienenden organischen Lösungsmittel durchgeführten Polymerisation erhaltene Filtrat verwendet wird und worin die Filtration solcherweise durchgeführt wird, daß Polymerteilchen im Filtrat als Polymerisationssaat verbleiben.

## Revendications

1. Procédé de fabrication d'un polymère acrylique super-absorbant sous la forme de granules écoulables, caractérisé en ce que de l'acrylate de sodium et de l'acrylamide sont soumis à une polymérisation à un seul stade dans une suspension du type eau/huile dans un intervalle de températures de 40°C à 60°C, dans lequel, comme phase continue, on utilise un filtrat obtenu après séparation par filtration du polymère de la polymérisation précitée réalisée avec un solvant organique hydrophobe comme phase continue, et dans lequel la filtration est réalisée d'une manière telle que des particules de polymère restent dans le filtrat sous la forme de germes de polymérisation.
